# EUROPEAN PATENT SPECIFICATION

(11) **EP 3 298 279 B1**
(45) Date of publication and mention of the grant of the patent: **20.10.2021**
(21) Application number: 16745811.6
(22) Date of filing: 17.05.2016
(51) Int. Cl.: F04B 39/12, A61B 5/03, A61M 25/10, F04B 53/06

(54) **A CIRCUIT FOR FEEDING A FLUID TO AN INFLATABLE CHAMBER**
KREISLAUF ZUR FÖRDERUNG EINES FLUIDS IN EINE AUFBLASBARE KAMMER
CIRCUIT POUR ALIMENTER UNE CHAMBRE GONFLABLE AVEC UN FLUIDE

(30) Priority: 20.05.2015 IT UB20150841
(43) Date of publication of application: 28.03.2018
(73) Proprietor: THD S.p.A., 42015 Correggio, Reggio Emilia (IT)
(72) Inventor: BASTIA, Filippo, 41019 Soliera (Modena) (IT)
(74) Representative: Casadei, Giovanni
(86) International application number: PCT/IB2016/052856
(87) International publication number: WO 2016/185377

(56) References cited:
- EP-A1- 0 351 758
- US-A- 3 543 759
- US-A- 3 698 381
- US-A- 4 796 606
- US-A- 4 969 866

## Description

The invention relates to a circuit for feeding a fluid to an inflatable chamber.

In particular but not exclusively the circuit according to the present invention is suitable for feeding an inflatable chamber associated to a probe for manometric measurement of the anal conduit.

Relatively simple circuits from a structural viewpoint are currently available, which are intended for feeding an inflatable chamber, particularly a small-volume chamber, with a limited pressure fluid. Such circuits or similar circuits are described in documents US3698381, EP0351758, US4969866, US3543759 and US4796606.

These circuits comprise a micro-compressor, a solenoid valve interposed between the micro-compressor and the chamber, and at least one pressure detector.

Because the pressures brought into play are rather limited (in the approx. order of 150 mmHg that is 20000 Pa) as well as the volumes to be filled, and because the probes must be of relatively small size in order to be transportable, use is made of limited performance micro-compressors. In order to not excessively strain the micro-compressor, especially during initial phases, rather complex solenoid valves are employed, for the control of which likewise complex and expensive processors are required. The high cost of the components necessary for a proper functioning of the micro-processor, results in a corresponding high cost of using the probes, which often hinders deployment and use thereof.

In addition, because the processor and the solenoid valve are structured in a complex way, this results in higher risks of malfunctioning and errors of the former.

It is an object of the present invention to provide a circuit for feeding a fluid to an inflatable chamber which allows to overcome the drawbacks of the currently available circuits.

An advantage of the circuit according to the present invention, as defined by the subject-matter of patent claim 1, is that it is considerably more simple and cost-saving than currently available circuits.

A further advantage of the circuit according to the present invention is that it is very reliable and accurate.

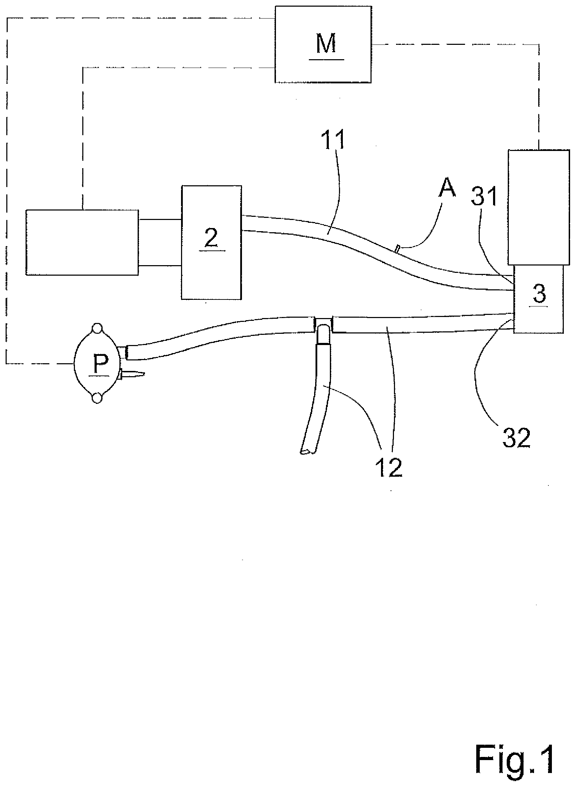

Further characteristics and advantages of the present invention will better emerge from the detailed description that follows of a preferred embodiment of the invention herein disclosed, illustrated by way of nonlimiting example in the appended figure 1.

The circuit according to the present invention comprises a compressor (2), preferably a micro-compressor. Since the compressor (2) is a device which is widely known to the man skilled in the art, it will not be described in further detail.

The circuit further comprises a two-way and two-position solenoid valve (3). The solenoid valve (3) is provided with a first port (31), which is connected to the compressor (2), and a second port (32) connectable to an inflatable chamber.

The solenoid valve (3) is operable between an open configuration, in which the ports (31,32) are in communication with one another, and a closed configuration, in which at least the second port (32) is closed. In a known way, the two open and closed configurations are obtained with a shaped obturator being made to slide by an electromagnetic actuator between two working positions. The above mentioned components are generally within the reach of the man skilled in the art and will therefore not be described in further detail.

A first conduit (11) connects the compressor (2) with the solenoid valve (3) at the first port (31). A second conduit (12) is connected to the solenoid valve (3) at the second port (32) and is predisposed for being connected to the inflatable chamber.

The circuit further comprises a pressure detector (P), which is arranged for measuring the pressure inside the second conduit (12). The pressure inside the second conduit (12) is in fact correspondent, or at least significant, of the pressure inside the inflatable chamber connected to the second conduit (12) itself. The pressure detector as well is known to the men skilled in the art and will not be further described.

A control module (M), for example in the form of micro-processor, or electronic circuit card, is arranged for receiving as input the pressure signal by the detector (P) and controlling actuation of the solenoid valve (3) and the compressor (2).

In particular, the control module (M) may be connected to an input device (not illustrated) via which it is possible to impart to the circuit at least one chamber inflation command and at least one chamber deflation command.

For example, in the case in which the inflatable chamber is associated with a probe for manometric measurement, the chamber inflation command corresponds to a start command of the measurement procedure, whereas the deflation command corresponds to a termination command of the measurement procedure.

The circuit according to the present invention comprises a vent opening (A) arranged along the first conduit (11). This vent opening (A) comprises, for example, a needle or cannulate pin inserted through the wall of the first conduit (11).

The presence of the vent opening (A) is extremely advantageous. First of all the vent opening (A) allows use of a solenoid valve (3) which is structured in a very simple manner i.e. a two-way and two-position solenoid valve. Indeed, in order to allow deflation of the chamber it is sufficient to bring and maintain the solenoid valve (3) in the open configuration, in that the fluid present inside the chamber can flow out through the second conduit (12), the solenoid valve (3) and the vent opening (A), without affecting the compressor. It is therefore not necessary to provide a discharge port for the solenoid valve (3) as occurs in the case of circuits of the known type.

To this end, the vent opening (A) is calibrated so as to allow discharge of the fluid from the second conduit (12) under stopping conditions of the compressor (2) as well as in the open configuration of the solenoid valve (3).

In addition, during startup of the compressor, the vent opening (A) allows to reduce the backpressure opposite to the compressor (2), which compressor (2) is consequently subject to a lower load. This is particularly useful because the motors generally used for the startup of compressors are of limited performance. The vent opening (A) is so calibrated as to allow feeding of the fluid from the compressor (2) to the second conduit (12) in the open configuration of the solenoid valve (3) in order to enable inflation of the chamber. In other words, the vent opening (A) is calibrated so as to allow discharge of a fluid flow rate lower than the overall flow rate which reaches the solenoid valve (3).

For the purposes of management and control of the circuit, the control module (M) is arranged to perform the following control steps, via proper software and/or firmware.

In the presence of an inflation or pressurization command of the inflatable chamber, or in general of a circuit start command, the control module (M) detects the pressure signal from the pressure detector (P).

If the pressure measured by the pressure detector (P) is lower than a certain threshold value, e.g. the pressure that one wishes to achieve and maintain within the inflatable chamber, then the control module (M) commands the opening of the solenoid valve (3) and the start of the compressor (2). If the pressure measured by the pressure detector (P) is greater than or equal to a certain threshold value, the control module (M) commands closing of the solenoid valve (3). The reading of the pressure measured by the pressure detector (P) takes place periodically at predetermined time intervals. If necessary, where the pressure measured by the pressure detector (P) is greater than or equal to a certain threshold value, then the control module (M) may be configured for commanding the stopping of the compressor (2).

In the presence of a deflation or depressurization command of the inflatable chamber, the control module (M) commands stopping of the compressor (2) and opening of the solenoid valve (3). As already noted, under such conditions the fluid within the inflatable chamber flows out through the second conduit (12), the solenoid valve (3) and the vent opening (A).

## Claims

1. A circuit for feeding a fluid to an inflatable chamber, comprising:
- a compressor (2);
- a two-way and two-position solenoid valve (3), provided with a first port (31) and a second port (32) and operable between an open configuration, in which the ports (31,32) are in communication with one another, and a closed configuration, wherein at least the second port (32) is closed;
- a first conduit (11) connecting the compressor (2) to the solenoid valve (3);
- a second conduit (12) connected to the solenoid valve (3) and predisposed for being connected to an inflatable chamber;
- a pressure detector (P), arranged for measuring the pressure inside the second conduit (12);
- a control module (M), predisposed for receiving a pressure signal from the pressure detector (P), and controlling the solenoid valve (3) as well as the compressor (2);
**characterized in that** it comprises a vent opening (A) arranged along the first conduit (11).

2. A circuit according to claim 1, wherein the vent opening (A) is calibrated so as to enable feeding of the fluid from the compressor (2) to the second conduit (12) in the open configuration of the solenoid valve (3).

3. A circuit according to claim 1, wherein the vent opening (A) is calibrated so as to enable discharge of the fluid from the second conduit (12) under stopping conditions of the compressor (2), as well as in the open configuration of the solenoid valve (3).

4. A circuit according to claim 1, wherein the control module (M) is predisposed for performing the following control steps:
- if a command for deflating or depressurizing the inflatable chamber is received, commanding the stopping of the compressor (2) and the opening of the solenoid valve (3).

5. A circuit according to claim 1, wherein the control module (M) is predisposed for performing the following control steps:
- if a command for inflating or pressurizing the inflatable chamber is received, detecting the pressure signal by the pressure detector (P);
- if the pressure measured by the pressure detector (P) is below a certain threshold value, commanding the start of the compressor (2) and the opening of the solenoid valve (3);
- if the pressure measured by the pressure detector (P) is greater than or equal to the given threshold value, commanding the closing of the solenoid valve (3).

6. A circuit according to claim 5, wherein the control module (M) is configured for commanding the stop of the compressor (2) if the pressure measured by the pressure detector (P) is greater than or equal to the certain threshold value.

## Patentansprüche

1. Kreislauf zur Förderung eines Fluids in eine aufblasbare Kammer, umfassend:
- einen Kompressor (2);
- ein Zweiwege- und Zweistellungs-Magnetventil (3), das mit einem ersten Anschluss (31) und einem zweiten Anschluss (32) versehen ist und zwischen einer offenen Konfiguration, in der die Anschlüsse (31, 32) in Kommunikation miteinander stehen, und einer geschlossene Konfiguration, in der zumindest der zweite Anschluss (32) geschlossen ist, betätigbar ist;
- eine erste Leitung (11), die den Kompressor (2) mit dem Magnetventil (3) verbindet;
- eine zweite Leitung (12), die mit dem Magnetventil (3) verbunden und ausgelegt ist, um mit einer aufblasbaren Kammer verbunden zu werden;
- einen Druckdetektor (P), der zum Messen des Drucks innerhalb der zweiten Leitung (12) angeordnet ist;
- ein Steuermodul (M), das ausgelegt ist, um ein Drucksignal vom Druckdetektor (P) zu empfangen und das Magnetventil (3) sowie den Kompressor (2) zu steuern;
**dadurch gekennzeichnet, dass** er eine Entlüftungsöffnung (A) umfasst, die entlang der ersten Leitung (11) angeordnet ist.

2. Kreislauf nach Anspruch 1, wobei die Entlüftungsöffnung (A) kalibriert ist, um die Förderung des Fluids vom Kompressor (2) zu der zweiten Leitung (12) in der offenen Konfiguration des Magnetventils (3) zu ermöglichen.

3. Kreislauf nach Anspruch 1, wobei die Entlüftungsöffnung (A) kalibriert ist, um das Ablassen des Fluids aus der zweiten Leitung (12) unter Stoppbedingungen des Kompressors (2) sowie in der offenen Konfiguration des Magnetventils (3) zu ermöglichen.

4. Kreislauf nach Anspruch 1, wobei das Steuermodul (M) zum Ausführen der folgenden Steuerschritte ausgelegt ist:
- wenn ein Befehl zum Entleeren oder Druckentlasten der aufblasbaren Kammer empfangen wird, Befehlen das Stoppen des Kompressors (2) und das Öffnen des Magnetventils (3).

5. Kreislauf nach Anspruch 1, wobei das Steuermodul (M) zum Ausführen der folgenden Steuerschritte ausgelegt ist:
- wenn ein Befehl zum Aufblasen oder Unterdrucksetzen der aufblasbaren Kammer empfangen wird, Erfassen des Drucksignals durch den Druckdetektor (P);
- wenn der vom Druckdetektor (P) gemessene Druck unter einem bestimmten Schwellenwert liegt, Befehlen das Starten des Kompressors (2) und das Öffnen des Magnetventils (3);
- wenn der vom Druckdetektor (P) gemessene Druck größer oder gleich dem angegebenen Schwellenwert ist, Befehlen das Schließen des Magnetventils (3).

6. Kreislauf nach Anspruch 5, wobei das Steuermodul (M) zum Befehlen des Stoppens des Kompressors (2) konfiguriert ist, wenn der von dem Druckdetektor (P) gemessene Druck größer oder gleich dem bestimmten Schwellenwert ist.

## Revendications

1. Circuit pour alimenter une chambre gonflable avec un fluide, comprenant :
- un compresseur (2) ;
- une électrovanne (3) à deux voies et à deux positions, pourvue d'un premier orifice (31) et d'un second orifice (32) et pouvant fonctionner entre une configuration d'ouverture, dans laquelle les orifices (31, 32) sont en communication l'un avec l'autre, et une configuration de fermeture, dans laquelle au moins le second orifice (32) est fermé ;
- un premier conduit (11) reliant le compresseur (2) à l'électrovanne (3) ;
- un second conduit (12) relié à l'électrovanne (3) et prédisposé pour être relié à une chambre gonflable ;
- un détecteur de pression (P) disposé pour mesurer la pression à l'intérieur du second conduit (12) ;
- un module de commande (M) prédisposé pour recevoir un signal de pression du détecteur de pression (P) et pour commander l'électrovanne (3) ainsi que le compresseur (2) ;
**caractérisé en ce qu'**il comprend une ouverture de ventilation (A) disposée le long du premier conduit (11).

2. Circuit selon la revendication 1, dans lequel l'ouverture de ventilation (A) est calibrée de manière à permettre l'alimentation du fluide du compresseur (2) vers le second conduit (12) dans la configuration d'ouverture de l'électrovanne (3).

3. Circuit selon la revendication 1, dans lequel l'ouverture de ventilation (A) est calibrée de manière à permettre le refoulement du fluide du second conduit (12) dans des conditions d'arrêt du compresseur (2), ainsi que dans la configuration d'ouverture de l'électrovanne (3).

4. Circuit selon la revendication 1, dans lequel le module de commande (M) est prédisposé pour réaliser les étapes de commande suivantes :
- si une commande de dégonflage ou de mise hors pression de la chambre gonflable est reçue, commander l'arrêt du compresseur (2) et l'ouverture de l'électrovanne (3).

5. Circuit selon la revendication 1, dans lequel le module de commande (M) est prédisposé pour réaliser les étapes de commande suivantes :
- si une commande de gonflage ou de mise sous pression de la chambre gonflable est reçue, détecter le signal de pression par le détecteur de pression (P) ;
- si la pression mesurée par le détecteur de pression (P) est inférieure à une certaine valeur de seuil, commander le démarrage du compresseur (2) et l'ouverture de l'électrovanne (3) ;
- si la pression mesurée par le détecteur de pression (P) est supérieure ou égale à la valeur de seuil donnée, commander la fermeture de l'électrovanne (3).

6. Circuit selon la revendication 5, dans lequel le module de commande (M) est configuré pour commander l'arrêt du compresseur (2) si la pression mesurée par le détecteur de pression (P) est supérieure ou égale à la valeur de seuil donnée.
